(19) Europäisches Patentamt
European Patent Office
Office européen des brevets

(11) **EP 2 670 294 B1**

(12) **FASCICULE DE BREVET EUROPEEN**

(45) Date de publication et mention de la délivrance du brevet:
**05.08.2015 Bulletin 2015/32**

(21) Numéro de dépôt: **12703735.6**

(22) Date de dépôt: **27.01.2012**

(51) Int Cl.:
***A61B 3/12*** *(2006.01)* ***A61B 3/10*** *(2006.01)*

(86) Numéro de dépôt international:
**PCT/EP2012/051327**

(87) Numéro de publication internationale:
**WO 2012/104211 (09.08.2012 Gazette 2012/32)**

(54) **MÉTHODE ET DISPOSITIF D'IMAGERIE RÉTINIENNE A HAUTE RÉSOLUTION**

VERFAHREN UND VORRICHTUNG FÜR HOCHAUFLÖSENDE RETINABILDGEBUNG

METHOD AND DEVICE FOR HIGH-RESOLUTION RETINAL IMAGING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.02.2011 FR 1150760**

(43) Date de publication de la demande:
**11.12.2013 Bulletin 2013/50**

(73) Titulaire: **Imagine Eyes**
**91400 Orsay (FR)**

(72) Inventeurs:
• **LEVECQ, Xavier**
**91190 Gif sur Yvette (FR)**
• **LAMORY, Barbara**
**91120 Palaiseau (FR)**

(74) Mandataire: **Osha Liang**
**2, rue de la Paix**
**75002 Paris (FR)**

(56) Documents cités:
**US-A1- 2004 207 811     US-A1- 2007 258 045**

• **A ROORDA ET AL.,: "Adaptive optics scanning laser ophthalmoscopy", OPTICS EXPRESS, vol. 10, no. 9, 6 mai 2002 (2002-05-06), pages 405-412, XP002636223, cité dans la demande**
• **ZAWADZKI ET AL.,: "Adaptive-optics optical coherence tomography for high-resolution and high-speed 3D retinal in vivo imaging", OPTICS EXPRESS, vol. 13, no. 21, 17 octobre 2005 (2005-10-17), pages 8532-8546, XP002636224, cité dans la demande**

## Description

## Domaine technique de l'invention

La présente invention concerne une méthode et un dispositif d'imagerie rétinienne à haute résolution, compatible avec l'imagerie à l'échelle cellulaire.

## Etat de l'art

Il s'écoule plusieurs années aujourd'hui entre le début d'une maladie rétinienne et son diagnostic. En effet, les maladies rétiniennes en général se développent silencieusement en provoquant des lésions irréversibles avant que les premiers symptômes cliniques apparaissent. C'est le cas par exemple de la Dégénérescence Maculaire Liée à l'Age (ou DMLA) ou du glaucome, maladie s'attaquant aux fibres nerveuses de la rétine et pouvant provoquer la cécité du patient, et qui est généralement diagnostiquée lorsque la moitié des fibres nerveuses sont irrémédiablement détruites. Or les maladies rétiniennes peuvent être diagnostiquées dès les premières semaines si on peut imager la rétine à l'échelle cellulaire. En effet, les premiers effets des maladies rétiniennes atteignent les structures microscopiques de la rétine. Les microstructures atteintes par les 3 maladies rétiniennes les plus courantes et parmi les plus graves (DMLA, glaucome, rétinopathie diabétique) sont les photorécepteurs, dont les cônes, cellules photosensibles qui détectent la lumière et qui présentent une taille variant entre 2 et 5 $\mu$m, les micro capillaires de la rétine qui sont les plus petit vaisseaux du corps humain (environ 6 $\mu$m de diamètre), et les faisceaux de fibres nerveuses qui présentent quant à eux un diamètre d'environ 10 $\mu$m.

De nombreux laboratoires travaillent sur différentes technologies qui permettraient de faire de l'imagerie rétinienne avec une résolution cellulaire. Ces différentes technologies proposent des systèmes d'illumination et/ou détection de la rétine différents mais toutes mettent en oeuvre un système d'optique adaptative permettant de mesurer les défauts optiques de l'oeil et du système d'imagerie et de corriger les rayons lumineux issus de la rétine et incidents sur le système de détection pour gagner en résolution.

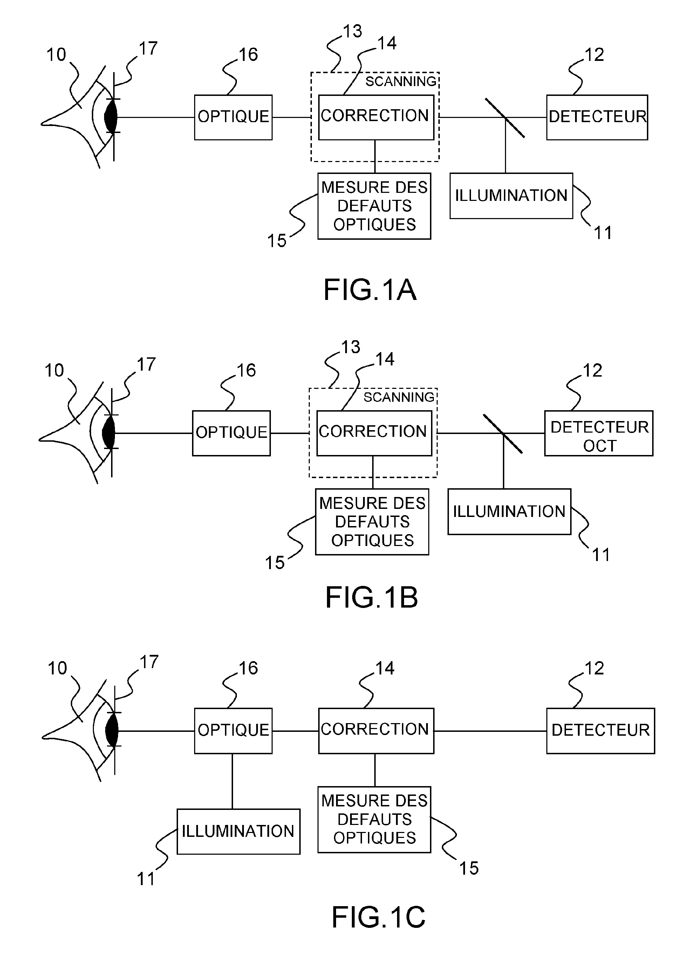

La figure 1A représente un schéma de principe d'un système d'imagerie rétinienne basée sur la technologie d'ophtalmoscopie à balayage laser avec optique adaptative ou 'AOSLO' (abréviation de l'expression anglo-saxonne 'Adaptive Optics Scanning Laser Ophtalmoscopy'). Le montage de l'AOSLO comprend principalement un système d'illumination 11 de la rétine ou «bloc illumination », un bloc détecteur 12, un bloc de balayage (« scanning ») 13, un système de correction 14 comprenant un plan de correction des rayons lumineux incidents, un système de mesure des défauts optiques 15 comprenant un plan d'analyse des défauts optiques de rayons lumineux incidents, et une optique d'imagerie 16. Le bloc illumination comprend par exemple une diode laser couplée à une fibre optique pour former un point source et une lentille optique permettant de former à partir du point source un faisceau d'éclairage. Un diaphragme du bloc illumination 11 définit une pupille. Le faisceau d'éclairage est envoyé, par exemple par un jeu de miroirs (non représentés), sur le système de correction 14, par exemple un miroir déformable, puis dans le bloc de balayage 13 pour être dirigé selon un balayage vertical et horizontal dans l'oeil 10 d'un sujet. Le faisceau d'éclairage est ainsi focalisé pour former sur la rétine un faisceau quasi ponctuel qui balaye la rétine et la lumière rétrodiffusée par la rétine subit le même balayage optique au retour pour être envoyée sur le miroir déformable 14 et le bloc détecteur 12, comprenant par exemple un trou de détection confocale et un détecteur qui peut être un photomultiplicateur ou une photodiode à avalanche. Un ensemble d'éléments optiques symbolisé par le bloc optique 16 participe à conjuguer optiquement le plan de la rétine et le trou de détection confocale du détecteur. Le système de mesure des défauts optiques 15 comprend par exemple un analyseur de type Shack-Hartmann ; il reçoit la lumière rétrodiffusée par la rétine et contrôle le miroir déformable afin de corriger le faisceau d'éclairage et le faisceau rétrodiffusé. Le plan de la pupille du bloc illumination, le plan du miroir déformable et le plan d'analyse du système de mesure des défauts optiques sont conjugués optiquement avec un plan prédéterminé 17 de l'oeil, par exemple le plan pupillaire de l'oeil. Le plan prédéterminé 17 est avantageusement le plan de la pupille d'entrée du système d'imagerie de la rétine sur le bloc détecteur. L'article de A. Roorda et al. (« Adaptive optics scanning laser ophtalmoscopy », Optics express 405, Vol. 10, N°9, 2002) décrit par exemple un dispositif tel que schématisé sur la figure 1A.

Dans la suite de la description, on entend par « défauts optiques » l'ensemble des perturbations que subissent les rayons lumineux entre la rétine et le détecteur. Ces défauts comprennent les défauts apportés par le système optique de l'oeil mais aussi par le système optique du système d'imagerie.

On entend par «pupille d'entrée » d'un système optique, la plus petite ouverture qui limite l'entrée ou la propagation des rayons lumineux dans le système. Cette ouverture peut être réelle dans le cas où un diaphragme physique, pupille du système optique considéré, limite l'entrée des rayons lumineux ou virtuelle dans le cas où cette ouverture est une image de la pupille physique du système optique qui se trouve à l'intérieur du système optique et qui est formé par exemple par un diaphragme. Ainsi, dans le cas où la pupille d'entrée du système d'imagerie 16 de la rétine est positionnée dans le plan pupillaire de l'oeil ou dans un plan se situant à proximité de ce dernier, ladite pupille d'entrée est virtuelle, image d'un diaphragme physique situé à l'intérieur dudit système optique d'imagerie.

La figure 1B représente un schéma de principe d'un montage de type OCT (abréviation de l'expression anglo-saxonne 'Optical Coherence Tomography') couplé à de l'optique adaptative. Un tel système est décrit par exemple dans R. Zawadzki (« Adaptive-optics optical coherence tomography for high resolution and high speed 3D retinal in vivo imaging » Optics Express 8532, Vol. 13, N°21, 2005). L'OCT repose sur l'utilisation d'un interféromètre à faible cohérence. Cette technique d'imagerie permet de réaliser in vivo des images en coupe de tissus, avec une résolution de quelques microns. Un intérêt de l'OCT en ophtalmologie provient de sa capacité à révéler in-vivo des tissus au travers d'autres tissus diffusants. Le montage de la figure 1B reprend de façon très simplifiée les éléments principaux d'un montage de type OCT. On retrouve un arrangement similaire à celui de l'AOSLO mais dans lequel le bloc détecteur 12 est spécifique à l'OCT et comprend notamment un interféromètre, par exemple un interféromètre fibré, par exemple de type Michelson. Le point d'entrée de la fibre (non représentée) est conjugué avec la rétine de l'oeil 10 au moyen d'un système optique de conjugaison symbolisé par l'optique 16. En comparaison avec l'AOSLO, la technologie OCT permet d'avoir une coupe longitudinale de la rétine au détriment de la vitesse d'acquisition.

La figure 1C représente un schéma de principe d'un système d'imagerie rétinienne plein champ ou 'flood' selon l'expression anglo-saxonne, décrit par exemple dans « Adaptive Optics Ophthalmoscopy » de A. Roorda (Journal of Refractive Surgery Vol.16 sep/oct 2000) ou dans H. Hofer et al. (« Improvements in retinal image quality with dynamic correction of the eye's aberrations », Optics Express, Vol. 8, Issue 11, pp. 631-643, 2001). Dans ce système, le bloc illumination comprend une première source d'illumination pour l'imagerie, étendue, et une seconde source d'illumination, ponctuelle, pour l'analyse des défauts optiques. Le bloc détecteur 12 comprend un dispositif d'acquisition multidétecteur (ou détecteur matriciel), par exemple une caméra CCD, dont le plan de détection est destiné à être conjugué optiquement avec la rétine de l'oeil 10 que l'on cherche à imager, grâce à un système optique de formation d'image - ou système d'imagerie - symbolisé par l'optique 16. Un système de mesure des défauts optiques 15, par exemple de type analyseur Shack-Hartmann, analyse les défauts optiques subis par les rayons issus de la source d'analyse et rétrodiffusés par la rétine. Il est relié à un système de correction 14, par exemple un miroir déformable, afin de corriger les rayons lumineux rétrodiffusés par la rétine. Comme dans les systèmes précédemment décrits, le plan d'analyse du système de mesure des défauts optiques et le plan du miroir déformable sont optiquement conjugués avec un plan prédéterminé de l'oeil, par exemple le plan pupillaire 17 de l'oeil, qui est avantageusement le plan de la pupille d'entrée du système d'imagerie de la rétine sur le détecteur 12. Le dispositif ainsi décrit en référence à la figure 1C, s'il est limité dans l'exploration en profondeur de la rétine, présente cependant par rapport aux systèmes de type OCT ou AOSLO l'avantage de fonctionner en plein champ, c'est-à-dire sans balayage mécanique de la rétine, et avec des temps d'acquisition de l'image complète beaucoup plus court, ce qui le rend à la fois moins complexe à réaliser, moins coûteux et moins sensible aux déformations que subit l'image pendant le temps d'acquisition, déformation qui sont générées par le mouvement de la rétine.

Dans chacun de ces dispositifs, un système d'imagerie 16 permet de former l'image de la rétine sur un bloc détecteur 12 adapté pour permettre la détection de structures de fréquences spatiales de l'ordre de 250 cycles/mm sur la rétine, formant une voie d'imagerie. Un dispositif de correction 14, par exemple un miroir déformable, comprenant un plan de correction des rayons lumineux rétrodiffusées par la rétine, contrôlé par un système de mesure des défauts optiques 15, permet de corriger tout ou partie des défauts optiques dus à l'oeil et au système optique du système d'imagerie et d'améliorer ainsi la qualité de l'image de la rétine formée sur le bloc de détection 12. Le système de mesure des défauts optiques permet de déterminer dans un plan d'analyse et en une mesure les défauts optiques d'une onde lumineuse incidente. Il s'agit avantageusement d'un analyseur de type Shack-Hartmann comprenant un plan d'analyse formé d'un ensemble de microlentilles et d'un détecteur matriciel agencé dans le plan focal desdites microlentilles. Dans ces systèmes, le plan d'analyse de l'analyseur des défauts optiques et le plan de correction du dispositif de correction sont conjugués optiquement avec un plan prédéterminé de l'espace d'entrée du système d'imagerie, plan réel destiné à être confondu avec un plan prédéterminé de l'oeil, par exemple le plan pupillaire de l'oeil. La pupille d'entrée du système d'imagerie est avantageusement située dans ce même plan prédéterminé. La voie d'analyse est ainsi formée du système de mesure des défauts optiques 15 et des moyens de conjugaison du plan d'analyse avec ledit plan prédéterminé dans l'espace d'entrée du système d'imagerie. La pupille d'entrée du système d'imagerie est par exemple une image de la pupille physique du dispositif de correction, formée par exemple par un diaphragme et définissant la surface utile du dispositif de correction.

Il est courant de chercher à travailler avec une taille de la pupille d'entrée du système d'imagerie entre la rétine et le détecteur la plus grande possible, à la fois pour gagner en résolution et pour rendre maximal le flux lumineux issu de la pupille de l'oeil et donc bénéficier d'un meilleur rapport signal sur bruit.
Le document US-A-2007/0258045 décrit un système d'imagerie rétinienne permettant de réaliser simultanément une image grand-champ et faible résolution de la rétine et une image petit-champ et haute résolution de la rétine.

La déposante a montré qu'au contraire de l'effet attendu, la limitation de la taille de la pupille dans une certaine mesure permettait une amélioration de la qualité de l'image en améliorant significativement le rapport signal sur bruit, ceci étant dû notamment à la nature de la lumière rétroréfléchie par la rétine.

RESUME DE L'INVENTION

Selon un premier aspect, l'invention concerne un dispositif d'imagerie rétinienne à haute résolution comprenant :

- au moins une source d'émission d'un faisceau lumineux pour l'illumination de la rétine d'un oeil d'un sujet, émettant dans une plage de longueurs d'onde d'imagerie donnée,
- une voie d'imagerie de la rétine comprenant un dispositif de détection apte à détecter des structures de fréquence spatiale de 250 cycles/mm mesurées dans le plan de la rétine et un système optique d'imagerie,
- une voie d'analyse comprenant un dispositif de mesure de défauts optiques avec un plan d'analyse , destiné à recevoir un ensemble de rayons lumineux rétrodiffusés par la rétine et des moyens de conjugaison optique dudit plan d'analyse avec un plan prédéterminé dans l'espace d'entrée dudit système d'imagerie de la voie d'imagerie,
- un dispositif de correction comprenant un plan de correction et destiné à corriger dans ledit plan de correction les rayons lumineux issus de ladite source d'émission et rétrodiffusés par la rétine en fonction des défauts optiques mesurés par le dispositif de mesure des défauts optiques, ledit plan de correction étant conjugué optiquement avec ledit plan prédéterminé dans l'espace d'entrée dudit système d'imagerie de la voie d'imagerie s.

Selon le premier aspect de l'invention, la pupille d'entrée du dudit système optique d'imagerie présente un diamètre compris entre une première valeur $\Phi_{min}$ et une seconde valeur $\Phi_{max}$, la première valeur étant définie pour permettre la détection par ledit dispositif de détection à la longueur d'onde centrale de ladite plage de longueurs d'onde d'imagerie de structures de la rétine présentant une fréquence spatiale de 250 cycles par millimètre, et la seconde valeur étant inférieure à 5,75 mm.

La déposante a montré notamment qu'en limitant le diamètre de la pupille d'entrée du système d'imagerie, et contrairement à l'idée reçue, le rapport signal sur bruit du dispositif de formation d'imagerie rétinienne se trouvait amélioré et le contraste des microstructures de la rétine de ce fait accru.

Avantageusement, la pupille d'entrée du système d'imagerie est positionnée dans ledit plan prédéterminé dans l'espace d'entrée du système d'imagerie, permettant une meilleure homogénéité de l'intensité lumineuse dans tout le champ de l'image.

Selon une variante, le dispositif de correction comprend un miroir déformable et la pupille du miroir déformable définit la pupille physique du système d'imagerie.

Avantageusement, la première valeur $\Phi_{min}$ est définie en fonction de ladite longueur d'onde centrale de la plage de longueurs d'onde d'imagerie pour obtenir un contraste théorique du système d'imagerie supérieur à 5% à ladite fréquence spatiale de 250 cycles par millimètre. La déposante a en effet démontré comment, dans les dispositifs d'imagerie rétinienne, la limitation du rapport signal sur bruit de la détection nécessitait une ouverture suffisante de l'optique pour permettre la détection de structures fines de la rétine.

Avantageusement, ladite première valeur $\Phi_{min}$ est donnée par la relation $\Phi_{min} = 5000 \times \lambda$, où $\lambda$ est la longueur d'onde centrale de la plage de longueurs d'onde d'imagerie d'imagerie.

La déposante a démontré que les valeurs optimales du diamètre de la pupille d'entrée du système d'imagerie dépendent de la plage de longueurs d'onde d'imagerie et qu'on peut donc définir des plages de valeurs en fonction de la plage de longueurs d'onde à l'intérieur desquelles le rapport signal sur bruit sera optimal et la résolution la meilleure, cela quel que soit le dispositif d'imagerie rétinienne haute résolution utilisée.

Selon une variante, la longueur d'onde centrale de la plage de longueurs d'onde d'imagerie est comprise entre 750 et 1100 nm et le diamètre de la pupille d'entrée du système d'imagerie est compris entre 3,75 mm et 5,75 mm. Selon une variante, la longueur d'onde centrale de la plage de longueurs d'onde d'imagerie est comprise entre 500 et 750 nm et le diamètre de la pupille d'entrée du système d'imagerie est compris entre 2,5 mm et 5,25 mm. Selon une variante, la longueur d'onde centrale de la plage de longueurs d'onde d'imagerie est comprise entre 350 et 500 nm et le diamètre de la pupille d'entrée du système d'imagerie est compris entre 1,75 mm et 4,25 mm.

Selon une variante, le dispositif est de type plein champ. La source d'illumination est alors une source étendue permettant d'illuminer la rétine avec un champ donné, et le dispositif de détection comprend un détecteur matriciel. Le dispositif comprend en outre une seconde source d'illumination de la rétine émettant dans une plage de longueurs d'onde d'analyse qui est avantageusement différente de plage de longueurs d'onde d'imagerie, ponctuelle, pour l'analyse des défauts optiques par ledit dispositif de mesure de défauts optiques.

Selon une autre variante, le dispositif est de type AOSLO. La source d'illumination est selon cette variante une source ponctuelle permettant d'illuminer la rétine avec un faisceau d'illumination quasi ponctuel et le dispositif de détection comprend un système de détection confocal. Le dispositif comprend en outre selon cette variante un système de balayage dudit faisceau d'illumination sur la rétine.

Selon une autre variante, le dispositif est de type OCT. La source d'illumination est une source ponctuelle permettant d'illuminer la rétine avec un faisceau d'illumination quasi ponctuel et le dispositif de détection comprend un interféromètre. Le dispositif comprend en outre selon cette variante un système de balayage dudit faisceau d'illumination sur la rétine.

Avantageusement, le dispositif de mesure des défauts optiques est un analyseur de type Shack-Hartmann. Un tel dispositif permet d'analyser, par rapport à des directions nominales, la variation des directions des rayons lumineux après avoir traversé le système optique entaché de défauts optiques. Un tel système réalise cette mesure grâce, par exemple, à l'agencement d'un détecteur matriciel dans le plan focal d'une matrice de microlentilles. Les variations ainsi mesurées peuvent être directement exploités pour le contrôle du dispositif de correction des défauts optiques.

Selon un second aspect, l'invention concerne une méthode d'imagerie rétinienne haute résolution, comprenant

- l'émission d'au moins un faisceau lumineux pour l'illumination de la rétine d'un oeil d'un sujet, dans une plage de longueurs d'onde d'imagerie donnée, au moyen d'une source d'émission lumineuse,
- la formation d'une image d'au moins une partie de la rétine illuminée par ledit faisceau lumineux émis dans ladite plage de longueurs d'onde d'imagerie sur un plan de détection d'un dispositif de détection apte à détecter des structures de fréquence spatiale de 250 cycles/mm mesurées dans le plan de la rétine et au moyen d'un système optique d'imagerie de pupille d'entrée de diamètre donné,
- la mesure de défauts optiques par l'analyse dans un plan d'analyse donné des défauts optiques de rayons lumineux rétrodiffusés par la rétine, ledit plan d'analyse étant conjugué avec un plan prédéterminé de l'oeil,
- la correction dans un plan de correction donné des rayons lumineux issus de ladite source d'émission et rétrodiffusés par la rétine en fonction des défauts optiques mesurés, ledit plan de correction étant conjugué optiquement avec ledit plan prédéterminé de l'oeil.

Selon le second aspect de l'invention, le diamètre de la pupille d'entrée du dudit système optique d'imagerie est compris entre une première valeur $\Phi_{min}$ et une seconde valeur $\Phi_{max}$, la première valeur étant définie pour permettre la détection par ledit dispositif de détection (12) à la longueur d'onde centrale de ladite plage de longueurs d'onde d'imagerie de structures de la rétine présentant une fréquence spatiale de 250 cycles par millimètre, et la seconde valeur étant inférieure à 5,75 mm.

Selon une variante, la méthode est une méthode d'imagerie rétinienne de type plein champ, comprenant en outre l'émission d'un faisceau lumineux d'analyse dans une plage de longueurs d'onde d'analyse pour l'analyse des défauts optiques, et dans laquelle le faisceau lumineux émis dans la plage de longueurs d'onde d'imagerie permet l'illumination de la rétine avec un champ donné et la formation de l'image dudit champ de la rétine est faite au moyen d'un détecteur matriciel.

Selon une variante, la méthode est de type AOSLO, comprenant en outre un balayage dudit faisceau d'illumination de la rétine et une détection confocale.

Selon une variante, la méthode est de type OCT, comprenant en outre une détection interférométrique.

BREVE DESCRIPTION DES DESSINS

D'autres avantages et caractéristiques de l'invention apparaîtront à la lecture de la description, illustrée par les figures suivantes :

- Figures 1A à 1C (déjà décrites), des schémas de principe de systèmes d'imagerie rétinienne connus de l'art antérieur ;

- Figure 2, une courbe montrant l'évolution théorique de la FTM en fonction de la fréquence à une longueur d'onde donnée (850 nm), pour deux diamètres de pupille;

- Figure 3A, une image de la rétine mesurée expérimentalement chez un sujet et Figure 3B une courbe montrant l'intensité mesurée sur trois photorécepteurs;

- Figure 4, une courbe montrant en fonction de la longueur d'onde le diamètre minimal de la pupille d'entrée du système d'imagerie nécessaire pour atteindre la résolution requise de 250 cycles par mm ;

- Figure 5, la courbe montrant la répartition de l'énergie lumineuse rétrodiffusée par la rétine en fonction de la position dans le plan de la pupille ;

- Figures 6A et 6B, des courbes montrant la variation du rapport signal sur bruit normalisé en fonction du diamètre de la pupille dans un dispositif d'imagerie rétinienne de type plein champ, respectivement dans le proche infra rouge (850 nm) et dans le visible (550 nm);

- Figure 7A, une courbe montrant dans un système d'imagerie rétinienne de type SLO le pourcentage de lumière provenant de la couche des photorécepteurs en fonction de la lumière totale rétrodiffusée par la rétine, en fonction du diamètre du trou de détection confocale ;

- Figure 7B, une courbe montrant la variation du rapport signal sur bruit normalisé en fonction du diamètre de la pupille dans un dispositif d'imagerie rétinienne de type SLO, dans le proche infra rouge (850 nm).

- Figures 8A à 8F des courbes montrant la variation du rapport signal sur bruit normalisé en fonction du diamètre de la pupille et dans des dispositifs d'imagerie rétinienne de type plein champ ou SLO, à différentes longueurs d'onde.

- Figure 9, un exemple de réalisation d'un système d'imagerie rétinienne de type plein champ, selon l'invention ;

- Figure 10A et 10B des images de rétines mesurées dans des systèmes d'imagerie rétinienne de type plein champ à 850 nm, respectivement avec une pupille de 7,5 mm et une pupille de 5 mm.

DESCRIPTION DETAILLEE

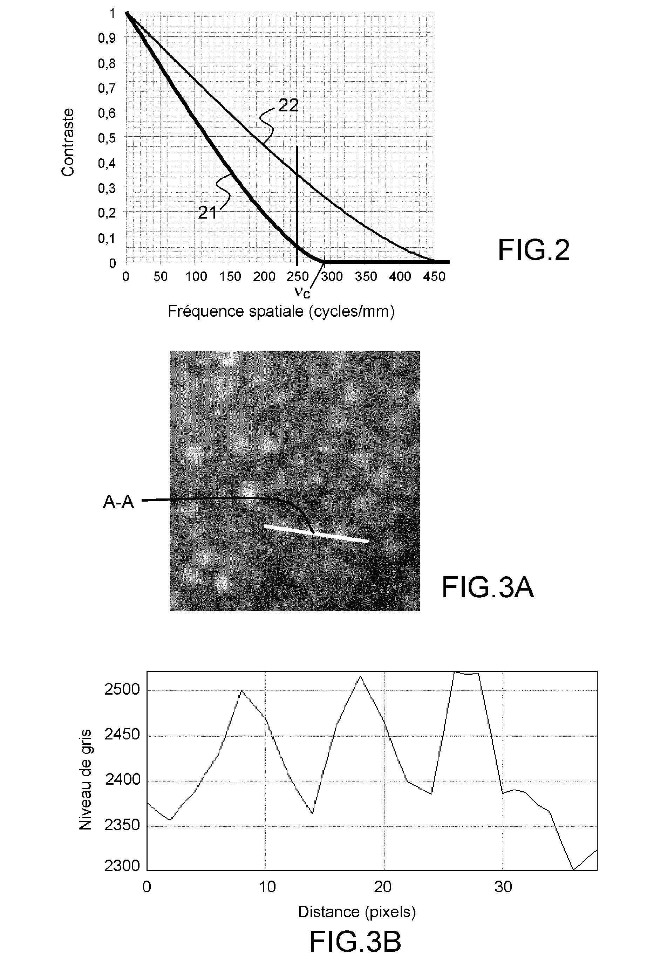

La figure 2 représente une courbe (référence 21) illustrant la fonction de transfert de modulation en fonction de la fréquence (donnée en cycles par millimètre) dans un système optique parfaitement corrigé limité par diffraction. La valeur $v_c$ est la fréquence de coupure, c'est-à-dire la fréquence à laquelle le contraste est nul. La fréquence de coupure $v_c$ est donnée par :

$$v_c = \frac{\Phi}{\lambda F} \qquad (1)$$

Où $\phi$ est le diamètre de la pupille d'entrée du système optique, F est la focale du système optique et $\lambda$ est la longueur d'onde.

Dans un système d'imagerie rétinienne du type de ceux décrits précédemment, on cherche à former l'image de structures de la rétine, par exemple des cônes, photorécepteurs dont la dimension à proximité du centre de la fovéa est de l'ordre de 2 $\mu$m et qui sont répartis en mosaïque de période spatiale de 4 $\mu$m environ. Détecter ces structures nécessite de pouvoir résoudre grâce au dispositif d'imagerie une fréquence spatiale de 250 cycles par millimètre sur la rétine. Si le rapport signal sur bruit du détecteur dans le système optique était infini, un diamètre minimal de la pupille nécessaire pour l'observation des cônes serait donné par l'équation (1) en prenant pour fréquence spatiale la fréquence correspondant aux éléments que l'on cherche à observer, soit 250 cycles par millimètre. Mais dans les dispositifs d'imagerie rétinienne, qu'ils soient de type OCT, AOSLO, plein champ, le rapport signal sur bruit est limité par le dispositif de détection et le flux rétrodiffusé par la rétine. Un diamètre minimal de la pupille d'entrée plus grand - correspondant à un meilleur contraste à la fréquence spatiale d'intérêt sur la courbe de la figure 2 - est donc nécessaire pour que le signal puisse être détecté.

Il est possible pour chacun des dispositifs d'imagerie rétinienne d'évaluer un rapport signal sur bruit, de façon théorique ou expérimentale, en fonction du dispositif de détection utilisé.

Un exemple de calcul réaliste dans l'état de l'art actuel du rapport signal sur bruit basé sur des données expérimentales est donné dans le cas d'un système d'imagerie de type plein champ, illustré par les figures 3A et 3B.

Dans la suite de la description, on parlera de « longueur d'onde » indifféremment soit pour la longueur d'onde d'une source d'émission lumineuse monochromatique, soit pour la longueur d'onde centrale d'une source d'émission lumineuse à spectre large, c'est à dire émettant dans une plage de longueur d'onde donnée.

L'illumination (11, figure 1C) de la rétine en vue de l'imagerie est réalisée par une source d'éclairage de type LED (diode électroluminescente) émettant des impulsions à 850 nm avec une largeur spectrale de 30 nm, de durée d'impulsion 9 ms et fréquence de récurrence 9,5 Hz. Le champ éclairé est de 4x4° soit 1.2x1.2 mm² environ sur la rétine. On envoie un flux de 0,12 mW moyen dans l'oeil, sur une pupille de 3 mm de diamètre. La densité d'énergie au niveau de la cornée est donc de 1,7 mW/cm². La caméra d'imagerie est une caméra CCD, 12 bits, présentant à 850 nm un rendement quantique de 0,2, un nombre d'électrons par niveau de 2,2 e-, un bruit de lecture de 8 e- et un niveau d'obscurité (dans le noir) de 150 niveaux. Dans ces conditions, une image (figure 3A) a été réalisée sur un oeil sain d'une personne de 45 ans. On y aperçoit un ensemble de points correspondant aux photorécepteurs sur la rétine. La figure 3B représente une coupe réalisée sur trois photorécepteurs de la rétine (ligne AA sur la figure 3A). Il est possible à partir de la figure 3B de calculer le rapport signal sur bruit donné par :

$$\frac{S}{B} = \frac{S_u}{\sqrt{S_t + B_l^2}} \qquad (2)$$

Où $S_u$ est le signal utile moyen, soit environ 150 niveaux (330 e-) si l'on se réfère à la figure 3B, $S_t$ est le signal total, égal à la somme du signal utile $S_u$ (330 e-) et du niveau moyen du signal détecté hors niveau de fond (2200 niveaux, soit 4840 e-) et $B_l$ est le bruit de lecture (8 e-). On calcule ainsi un rapport signal sur bruit de 4,6.

On peut alors extrapoler ce que serait le rapport signal sur bruit dans le cas d'une illumination plus importante. Dans les conditions d'utilisation de la caméra, le facteur qui limite la puissance d'illumination est lié à des considérations sur la sécurité oculaire. Plus précisément, le facteur qui doit être pris en compte dans l'hypothèse où le flux incident sur l'oeil est augmenté est la fluence sur la cornée. Dans les conditions de mesures ci dessus, la fluence sur la cornée est de 1,7 mW/cm$^2$ pour une limite permise à 20 mW/cm$^2$ pour les instruments de classe I (norme NF EN ISO 15004-2 2007 de sécurité oculaire). Dans l'hypothèse où l'on se place à la limite de la sécurité oculaire, on observe une augmentation de l'intensité lumineuse d'un facteur 11,8 et donc un rapport signal sur bruit multiplié par un facteur racine carrée de 11.8 (en négligeant le bruit de lecture qui est effectivement très négligeable comparé au bruit de photon du signal détecté), soit un facteur 3.43 ce qui amène le rapport signal à bruit à environ 16. La déposante a ainsi démontré que c'est une valeur du rapport signal sur bruit qui est atteignable ; Un tel rapport signal à bruit permet de détecter un objet dont le contraste ne serait que de 1/16 soit 6,25%.

En se rapportant maintenant à la figure 2, on peut en déduire un diamètre minimal de la pupille d'entrée du système, de telle sorte que pour la fréquence spatiale d'intérêt (250 cycles par millimètre), la FTM soit au moins égale à 6,25%. Cela correspond à une fréquence spatiale v = 0,85 $v_c$. La valeur minimale $\phi_{min}$ du diamètre de la pupille d'entrée du système est donc telle que :

$$\upsilon = 0{,}85\frac{\Phi_{min}}{\lambda f} = 250 \qquad (3)$$

où f est la focale de l'oeil mesurée dans l'air (soit 17mm) et λ la longueur d'onde de travail.

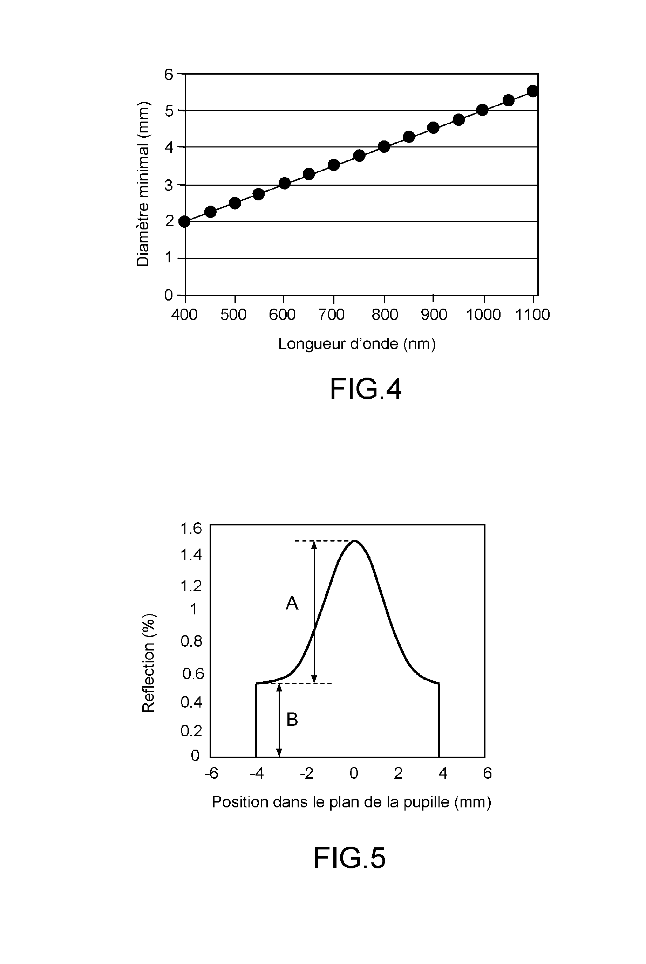

La figure 4 illustre le diamètre minimal de la pupille d'entrée du système en fonction de la longueur d'onde, selon l'équation 3 obtenue avec les paramètres calculés selon les hypothèses décrites ci-dessus. On peut déduire de cette courbe pour chaque longueur d'onde, la valeur minimale du diamètre de la pupille d'entrée pour pouvoir distinguer un élément de la rétine à la fréquence spatiale d'intérêt. Par exemple à 850 nm, qui est une longueur d'onde classiquement utilisée en imagerie rétinienne pour des questions de confort du sujet, le diamètre minimal de la pupille d'entrée est de 4,25 mm. De façon générale, en remplaçant dans l'équation (3) la focale f de l'oeil par sa valeur mesurée dans l'air (17 mm), on obtient ainsi dans cet exemple $\Phi_{min}$ = 5000 × λ.

Un calcul du rapport signal sur bruit maximal lié au dispositif de détection peut être fait dans les autres systèmes d'imagerie rétinienne. Ainsi par exemple, dans un système de type SLO, il a été démontré que le rapport signal sur bruit pouvait atteindre des valeurs de 10 à 15 en fonction de la taille du trou confocal, soit du même ordre de grandeur que celui atteint avec les systèmes de type plein champ.

Quelle que soit la technique d'imagerie, la déposante a ainsi démontré qu'il était réaliste de dimensionner la pupille d'entrée du système d'imagerie en choisissant un diamètre minimal tel que le contraste théorique obtenu soit supérieur à 5%, correspondant à un rapport signal sur bruit de la chaîne de détection du système inférieur à 20.

Quelle que soit la technique d'imagerie choisie, il est connu que le choix de diamètres de pupille d'entrée plus grands permet théoriquement de visualiser les structures de la rétine avec un meilleur contraste. Non seulement parce qu'en augmentant le flux utile, on augmente théoriquement la valeur du rapport signal sur bruit, mais aussi parce qu'avec une pupille d'entrée plus grande, la réponse de l'optique aux fréquences spatiales élevées est meilleure. Ainsi, si l'on se réfère à la figure 2, et si on reste dans l'hypothèse d'une longueur d'onde de travail à 850 nm, le choix d'une pupille de 7 mm de diamètre comparée à une pupille de diamètre 4.25 mm permettrait d'augmenter la fréquence de coupure et donc la valeur du contraste théorique d'un facteur proche de 6 (on passe de 6 à 35% de contraste) pour la fréquence de 250 cycles/mm (courbe 22 sur la figure 2).

Contrairement à cette première analyse, la déposante a montré tant de façon théorique qu'expérimentale, qu'il existait une valeur maximale de la taille de la pupille d'entrée au-delà de laquelle le rapport signal sur bruit se dégradait, et avec lui le contraste et la résolution du système.

Une première raison mise en évidence par la déposante pour expliquer la détérioration du rapport signal sur bruit est l'effet Stiles-Crawford, décrit par exemple dans l'article de Jan van de Kraats et Dirk van Norren (« Directional and nondirectional specral reflection from the human fovea », Journal of Biomedical Optics 13(2), 024010 (March/April 2008).

Cet article décrit le comportement directionnel de la couche des photorécepteurs de la rétine. Le signal rétrodiffusé par la rétine possède une composante non directionnelle issue des couches de la rétine située en amont et en aval de la couche des photorécepteurs et possède une composante directionnelle issue de la couche des photorécepteurs. Il ressort ainsi que la composante non directionnelle ne véhicule pas le signal utile (elle constitue principalement le bruit) ; de plus elle évolue avec la surface de la pupille utile, donc avec le carré du diamètre de la pupille. La composante directionnelle issue de la couche des photorécepteurs constitue le signal utile ; sa répartition d'énergie dans la pupille présente une forme gaussienne. De ce fait, la composante directionnelle n'évolue pas aussi vite que la composante non directionnelle lorsque la pupille varie. Il s'ensuit comme cela est décrit plus en détails ci-dessous que lorsque la pupille augmente, la composante non directionnelle liée au bruit augmente plus vite que la composante directionnelle (le signal). La figure 5, extraite de l'article de Jan van de Kraats et al., montre la contribution directionnelle (A) et non directionnelle (B) de la lumière rétrodiffusée par la rétine mesurée au niveau de la pupille de l'oeil.

La détérioration du rapport signal sur bruit est tout d'abord expliquée en référence à un système d'imagerie rétinienne de type plein champ tel qu'illustré sur la figure 1C.

L'expression de la répartition de l'énergie E(r) rétrodiffusée par la rétine dans la pupille est approximée par:

$$E(r) = B + A \exp(-2{,}3yr^2) \qquad (4)$$

Où B est l'amplitude de la composante non directionnelle (dépendante de la longueur d'onde), A est l'amplitude de la composante directionnelle et dépend également de la longueur d'onde et y est le coefficient de « directionnalité » et est dépendant de la longueur d'onde selon la formule :

$$y = 0{,}05 + 0{,}097 \times \left(\frac{500}{\lambda}\right)^2 \qquad (5)$$

Le signal intégré sur la pupille est donc, pour la composante non directionnelle (CND):

$$CND = B \times \pi \times r_{pup}{}^2 \qquad (6)$$

où $r_{pup}$ est le rayon de la pupille.

Et pour la composante directionnelle CD qui constitue le signal :

$$\int_0^{2\pi} \int_0^{r_{pup}} A e^{-2.3*y*r^2} \, r.dr.d\theta = \frac{A.\pi}{2.3*y}\left(1 - e^{-2.3*y*r_{pup}^2}\right)$$

Le rapport signal sur bruit SNR est donné par :

$$SNR = \frac{CD}{\sqrt{CND + CD}}$$

soit l'équation (7) ci-dessous :

$$SNR = \frac{A\sqrt{\pi}}{2.3*y*\sqrt{B}} * \frac{1 - e^{-2.3*y*r_{pup}^2}}{\sqrt{r_{pup}^2 + \frac{A}{B*2.3*y}\left(1 - e^{-2.3*y*r_{pup}^2}\right)}}$$

Le rapport signal sur bruit dépend donc de $r_{pup}$. Pour une longueur d'onde fixée, le rapport A/B est fixé. L'équation (7) montre que l'évolution, en fonction de $r_{pup}$, du rapport signal sur bruit normalisé ne dépend pas de A ni de B mais uniquement du rapport A/B.

Pour une longueur d'onde de 850 nm, on peut montrer que y=0,09 et A/B=0,1 (cette valeur ressort de l'article de Jan van de Kraats et al. et elle est confirmée par l'expérience).

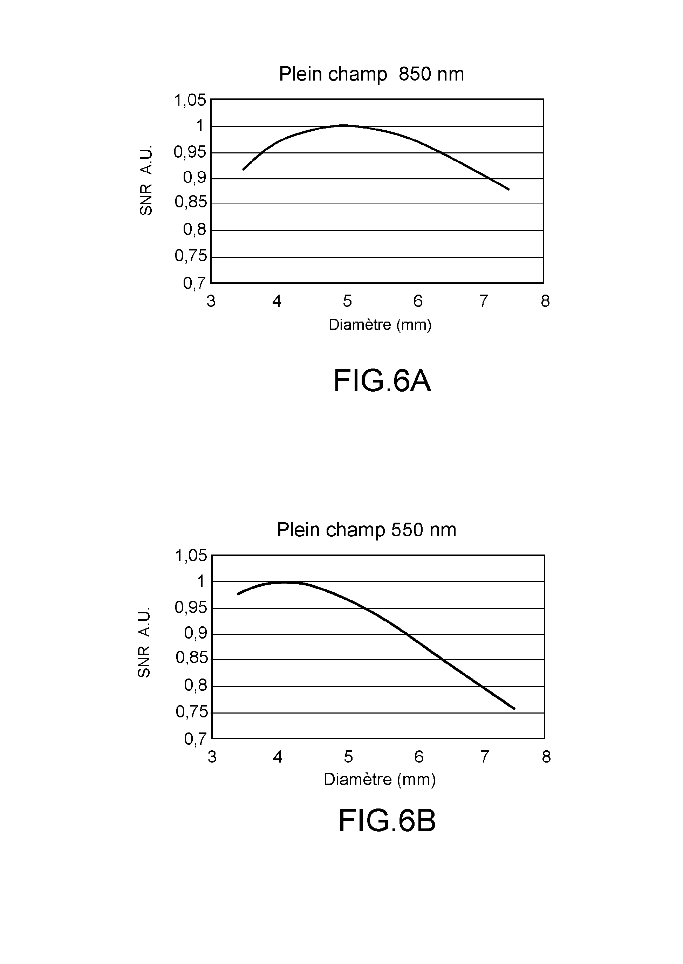

La courbe normalisée du rapport signal sur bruit ainsi obtenue est illustrée sur la figure 6A. On observe sur cette courbe que le rapport signal sur bruit, contrairement à l'idée reçue, passe par une valeur maximale du diamètre de la pupille d'entrée autour de 5 mm au-delà de laquelle il décroît.

Pour une longueur d'onde de 550 nm, y=0,147 et A/B=0,5 environ (là encore, cette valeur ressort de l'article de Jan van de Kraats et al et peut être confirmée par l'expérience). La courbe normalisée du rapport signal sur bruit ainsi obtenue est illustrée sur la figure 6B. Là encore, on observe une décroissance du rapport signal sur bruit lorsque le diamètre de la pupille croît au-delà de 4 mm environ.

L'effet du diamètre de la pupille peut être mis en évidence de la même façon dans le cas de systèmes d'imagerie rétinienne de type OCT ou AOSLO.

L'expression de la répartition de l'énergie rétrodiffusée par la rétine dans la pupille est donnée par l'équation (4) ci-dessus. Cependant, l'effet confocal dans les techniques OCT ou SLO réduit notablement au niveau du détecteur la transmission de la composante non directionnelle du flux rétrodiffusé par la rétine car les couches de la rétine qui rétrodiffusent cette composante sont situés au dessus ou en dessous de la couche des photorécepteurs qui est la couche mise au point sur le plan du trou du système confocal.

Le rapport signal sur bruit du flux rétrodiffusé par la rétine s'exprime de la même manière que dans le cas du système plein champ, à savoir il est donné par l'équation (7) ci-dessus. La différence réside dans le rapport A/B.

Pour évaluer la réflectance différentielle des couches rétiniennes on peut par exemple utiliser un système OCT commercial qui permet d'obtenir l'information du taux de réflectance de chaque couche de la rétine. Une fois que l'on a cette information on calcule quel est l'effet confocal en fonction de la taille du trou du système confocal (trou où une entrée de fibre). Une fois ce diamètre du trou défini, on détermine l'angle solide sous lequel est vue l'image du trou confocal par chacune des couches de la rétine ($\Omega_{pinhole}$) et on calcule ensuite l'angle solide sous lequel est vue la pupille de l'oeil par chacune des couches de la rétine ($\Omega_{pupille}$). L'angle solide limitant l'arrivée du flux lumineux provenant de chacune des couches de la rétine sera le plus petit de ces deux angles solides.

La valeur de ces angles solides dépend de la profondeur z de la couche. Par convention, on prendra z = 0 au niveau de la couche des photorécepteurs. Pour calculer les angles solides, on utilisera la relation suivante, permettant de calculer l'angle solide sous lequel est vu un disque de rayon R à une distance d du point d'observation :

$$\Omega = 2\pi \left( 1 - \frac{d}{\sqrt{d^2 + R^2}} \right)$$

On peut donc exprimer $\Omega_{pinhole}$ et $\Omega_{pupille}$ en fonction de z, du diamètre $\Phi_{pinhole}$ du trou confocal, du diamètre $\Phi_{pupille}$ de la pupille et de la focale f de l'oeil ramenée dans l'air (17 mm):

$$\Omega_{pinhole} = 2\pi \left( 1 - \frac{z}{\sqrt{z^2 + \frac{\emptyset^2_{pinhole}}{4}}} \right)$$

$$\Omega_{pupille} = 2\pi \left( 1 - \frac{f + z}{\sqrt{(f + z)^2 + \frac{\emptyset^2_{pupille}}{4}}} \right)$$

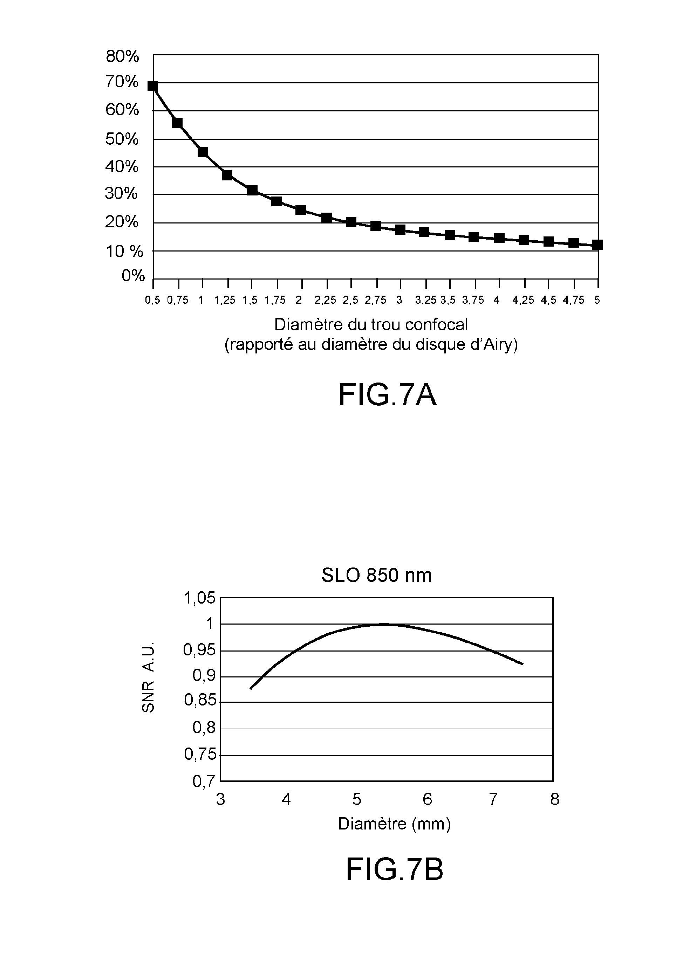

Il est alors possible de calculer l'angle solide de travail du système confocal égal au minimum des angles solides $\Omega_{pinhole}(z)$ et $\Omega_{pupille}(z)$ pour chacune des couches de la rétine visible sur le profil OCT (ou z est la distance entre la couche des photorécepteurs et la couche considérée). En combinant cette information avec l'information du taux de réflectance de chaque couche de la rétine on obtient une courbe qui donne le pourcentage de flux rétrodiffusé par la couche des photorécepteurs par rapport au flux total détecté en fonction de la taille du trou confocal (exprimé en nombre de fois la limite de diffraction). Une telle courbe est représentée sur la figure 7A. Cette courbe permet d'avoir accès directement au rapport A/B qui est dimensionnant pour déterminer l'évolution du rapport signal sur bruit normalisé. Si l'on considère le cas le plus utilisé aujourd'hui dans les système SLO où le diamètre du trou du système confocal est sensiblement égal au diamètre du la tache d'Airy, on obtient un rapport A/(A+B) = 0,45 ce qui implique que A/B = 0,82.

La courbe normalisée du rapport signal sur bruit ainsi obtenue pour un système AOSLO à 850 nm est illustrée sur la figure 7B (y=0,09 et A/B=0,82) . On observe une décroissance du rapport signal sur bruit à 850 nm lorsque le diamètre de la pupille croît au-delà de 5,5 mm environ.

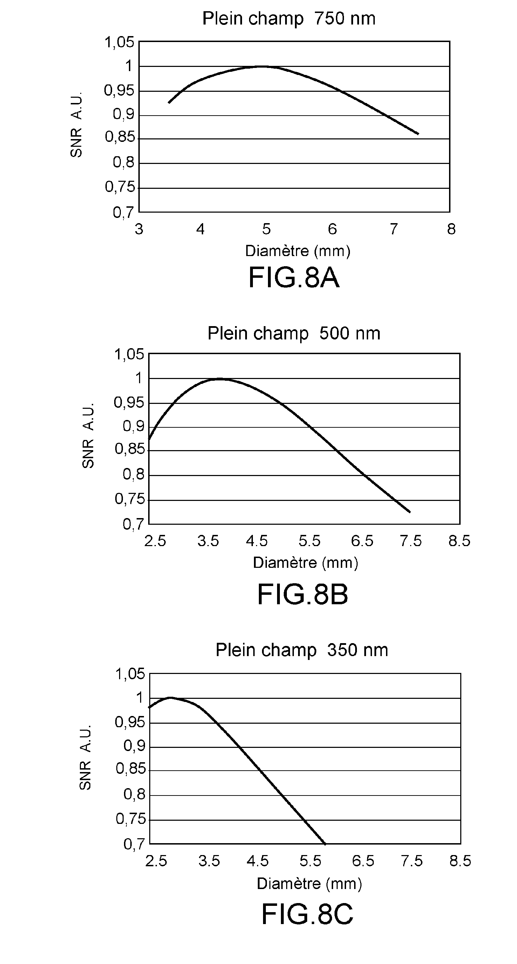

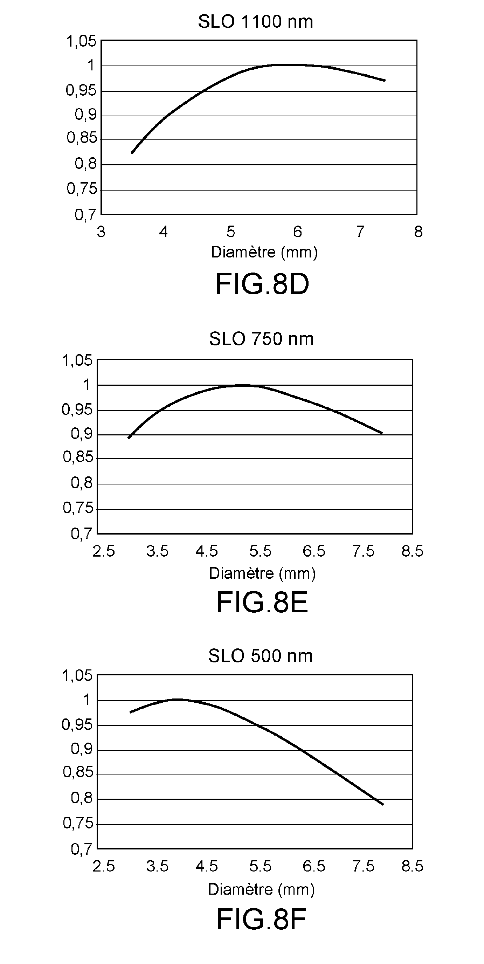

La déposante a ainsi déterminé les courbes donnant en fonction de la valeur du diamètre de la pupille d'entrée du système d'imagerie la valeur normalisée du rapport signal sur bruit, dans le cas de dispositifs d'imagerie rétinienne plein champ (figure 8A à 8B) et SLO (figures 8D à 8F). Ces courbes sont déterminées pour différentes longueurs d'onde d'imagerie, respectivement 750 nm, 500 nm et 350 nm pour les courbes 8A à 8C et 1100 nm, 750 nm et 500 nm pour les courbes 8D à 8F. Ces courbes mettent en évidence la valeur du diamètre de la pupille d'entrée à partir de laquelle le rapport signal sur bruit normalisé diminue.

Ainsi quelle que soit la technique utilisée, la déposante a montré l'apparition d'une dégradation du rapport signal sur bruit au-delà d'un diamètre de la pupille d'entrée dont la valeur dépend de la longueur d'onde. Typiquement, dans le proche infrarouge (entre 750 nm et 1100nm), le rapport signal sur bruit commence à se dégrader pour des diamètres de pupille supérieurs à des valeurs comprises entre 5 et 6 mm. Dans le visible (entre 500 nm et 750 nm), le rapport signal sur bruit commence à se dégrader pour des diamètres de pupille supérieurs à des valeurs comprises entre 4 et 5,25 mm. Dans le domaine spectral « bleu » (entre 350 nm et 500 nm) le rapport signal sur bruit commence à se dégrader pour des diamètres de pupille supérieurs à des valeurs comprises entre 3 et 4,25 mm.

Une seconde raison mise en évidence par la déposante pour expliquer la détérioration du rapport signal sur bruit est la présence chez un nombre important de sujets, et notamment de sujets âgés qui sont les plus touchés par les maladies rétiniennes, d'implants intra oculaires. L'intervention chirurgicale pour le traitement de la cataracte consiste en effet à enlever le cristallin opaque, et le remplacer par un cristallin artificiel (implant intra-oculaire) qui prend place dans l'« enveloppe » du cristallin (appelée capsule) laissée partiellement en place pendant l'intervention (*extraction extra capsulaire*). En termes chirurgicaux, l'opération d'une cataracte sénile comprend donc l'extraction extra capsulaire du cristallin latéralisé (droit ou gauche) par phacoémulsification par ultrasons avec conservation de la capsule postérieure et mise en place d'un implant intra capsulaire. La taille utile de l'implant intra oculaire est limitée par le contour du trou circulaire réalisé dans la capsule (capsulo-rhexis) dont le diamètre est au maximum de 5 mm. Ramenée dans l'espace de sortie de l'oeil (c'est-à-dire en tenant compte du grandissement apporté par la cornée), qui est aussi l'espace d'entrée du système d'imagerie, la taille utile maximale est de 5,75 mm.

Ainsi, il apparaît qu'une limitation à 5,75 mm de la pupille d'entrée du système d'imagerie est avantageuse pour la qualité de l'image et ce, quelle que soit la longueur d'onde utilisée pour l'illumination de la rétine. En effet, tout rayon arrivant en dehors de ce diamètre de 5,75 mm sera bloqué quelque soit sa longueur d'onde.

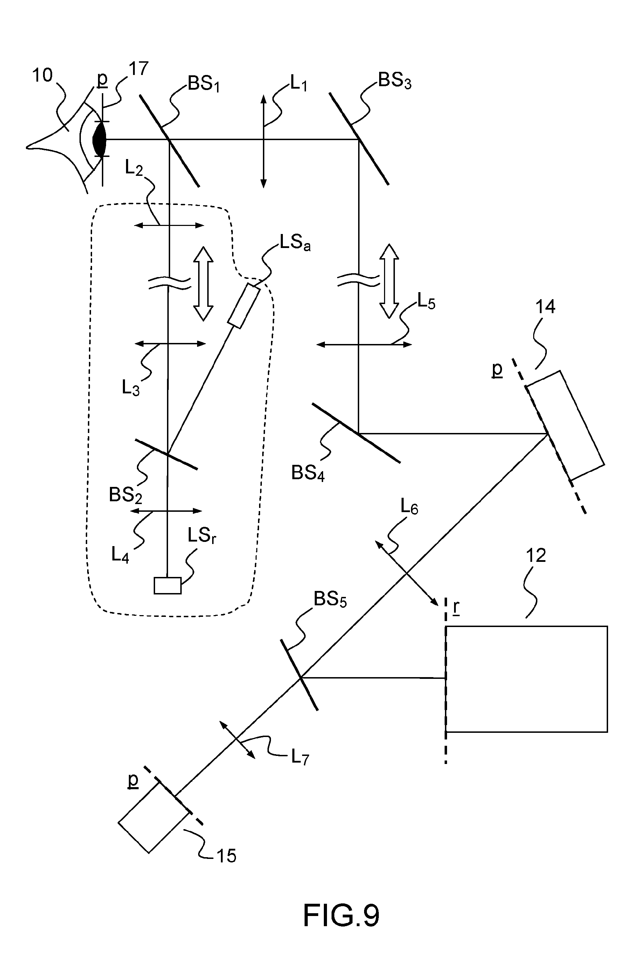

A titre illustratif, la figure 9 représente un exemple de dispositif d'imagerie rétinienne à haute résolution selon un exemple de réalisation de l'invention basé sur la technologie plein champ autrement appelée 'flood'. Sur la figure 9, seuls les éléments du dispositif nécessaires à la compréhension de l'invention sont représentés. Le dispositif d'imagerie comprend un bloc illumination 11, avec une première source $LS_r$ d'émission d'un faisceau lumineux destiné à éclairer la rétine d'un oeil 10 d'un sujet pour en former une image au moyen du bloc détecteur 12. Cette source est étendue, permettant d'éclairer la rétine de l'oeil avec un champ donné, typiquement 4° x 4° pour former une image dite « plein champ ». Avantageusement, la source d'illumination de la rétine $LS_r$ présente une longueur d'onde dans le proche infra rouge, typiquement entre 750 et 1100 nm, plage de longueurs d'onde présentant pour le sujet un confort oculaire plus grand et pour laquelle la longueur de pénétration dans les couches de la rétine est plus importante. Selon une variante, la longueur d'onde de la source d'illumination de la rétine $LS_r$ peut également être dans le visible pour réaliser des images en couleur de la rétine. Des longueurs d'onde dans le bleu, typiquement entre 350 et 500 nm, peuvent également être utilisées pour la visualisation des faisceaux de fibres nerveuses dans le cade du glaucome par exemple. La source $LS_r$ est par exemple une LED ou une lampe munie d'un filtre. Le bloc illumination 11 comprend également une seconde source $LS_a$ d'illumination de la rétine destinée à l'analyse des défauts optiques du système d'imagerie. Contrairement à la source d'illumination $LS_r$ destinée à l'imagerie, la source d'illumination $LS_a$ est une source ponctuelle, permettant de former un point source secondaire sur la rétine de l'oeil du sujet. Typiquement, la longueur d'onde centrale de la source d'illumination $LS_a$ pour l'analyse des défauts optiques est de 750 nm. Une telle longueur d'onde est confortable pour le sujet et aussi proche que possible de la longueur d'onde d'imagerie. De préférence la longueur d'onde de la source LSa est différente de celle de la source LSr pour des raisons de séparation des chemins optiques entre la mesure des défauts optiques et l'imagerie de la rétine. La source $LS_a$ est par exemple une diode laser ou diode superluminescente SLED. Un ensemble de lames séparatrices $BS_1$, $BS_2$, permet d'envoyer vers l'oeil 10 du sujet les faisceaux lumineux émis par les sources $LS_r$ et $LS_a$. Un ensemble d'éléments optiques $L_2$, $L_3$, $L_4$, sont utilisées pour former à partir des sources d'illumination des faisceaux collimatés incidents sur la pupille de l'oeil. L'image de la rétine est formée sur le bloc détecteur 12, comprenant par exemple une caméra d'imagerie de type CCD, au moyen d'un système d'imagerie comprenant notamment un ensemble d'éléments optiques référencés $L_1$, $L_5$, $L_6$ sur la figure 9. Le système d'imagerie présente une pupille d'entrée destinée à être positionnée dans un plan prédéterminé 17 de l'oeil, par exemple le plan pupillaire. Sur la figure 9, les plans référencés par la lettre « r » correspondent aux plans conjugués optiquement avec le plan de la rétine, tandis que les plans référencés par la lettre « p » correspondent aux plans conjugués optiquement avec ledit plan prédéterminé 17. Le dispositif d'imagerie rétinienne comprend en outre un dispositif 15 d'analyse des défauts optiques. Il s'agit d'analyser l'ensemble des perturbations que subissent les rayons lumineux entre la rétine et le détecteur. Les défauts optiques au sens de cette description comprennent donc les défauts apportés par le système optique de l'oeil mais aussi par la partie du système optique d'imagerie commune avec la voie d'analyse. Le dispositif d'analyse des défauts optiques est par exemple un analyseur de type Shack-Hartmann (HASO® 32-eye Imagine Eyes®), comprenant un plan d'analyse formé d'un ensemble de micro lentilles et un détecteur disposé dans le plan focal des micro lentilles. Le plan d'analyse est avantageusement conjugué optiquement avec le plan 17 de la pupille d'entrée du système d'imagerie au moyen des éléments optiques $L_1$, $L_5$, $L_6$ et d'un élément optique supplémentaire $L_7$. Un calculateur (non représenté) permet de déterminer les défauts optiques du système et d'envoyer une commande de correction au dispositif de correction 14, par exemple un miroir déformable de type mirao 52-e Imagine Eyes®. Avantageusement, le calculateur, associé au Shack-Hartmann, détermine par rapport à des directions nominales la variation des directions des rayons lumineux après avoir traversé le système optique entaché de défauts optiques. Les variations ainsi mesurées peuvent être directement exploités pour le contrôle du miroir déformable. Le plan du miroir déformable est également conjugué optiquement avec le plan 17 de la pupille d'entrée du système d'imagerie. Un ensemble de lames séparatrices, référencées $BS_4$, $BS_5$, $BS_6$, sur la figure 9, permettent de diriger les rayons lumineux issus des sources d'illumination $LS_r$ et $LS_a$ et rétrodiffusés par la rétine sur le miroir déformable 12 puis respectivement sur le détecteur 12 et l'analyseur 15, formant respectivement les faisceaux d'imagerie et d'analyse. Selon une variante, la pupille d'entrée 17 du dispositif d'imagerie est une image de la pupille du miroir déformable.

Une étude clinique a été menée pour vérifier expérimentalement l'amélioration de la qualité de l'image avec l'optimisation de la taille de la pupille d'entrée du système d'imagerie. Le protocole mis en place est basé entre autre sur la mesure de la rétine avec 2 dispositifs d'imagerie rétinienne à haute résolution de type plein champ intégrant un système d'optique adaptative. Ces dispositifs sont du type de ceux décrits sur la figure 9 mais l'un des deux dispositifs présente une pupille d'entrée de 7,5 mm de diamètre, l'autre une pupille d'entrée de 5 mm. La longueur d'onde d'imagerie est de 850 nm dans les deux dispositifs. L'architecture globale ainsi que tous les composants sont identiques (mêmes caractéristiques) pour les 2 dispositifs. Dans les deux cas la pupille du composant correcteur, en l'occurrence un miroir déformable mirao 52e (Imagine Eyes®), représente la pupille physique du système d'imagerie. La seule différence entre les deux dispositifs réside donc dans le grandissement optique entre la pupille du miroir déformable et l'oeil. Dans le cas du système de diamètre de pupille 7,5 mm, le grandissement est de 2 entre la pupille de l'oeil et le miroir déformable (de diamètre de pupille 15 mm) et dans le cas du système de diamètre de pupille d'entrée de 5 mm, le grandissement est de 3.

Le nombre d'yeux imagés dans le cadre de cette étude est de 19. Pour chaque oeil, 3 images sont réalisées au niveau de la couche des photorécepteurs pour 2 degrés et 5 degrés d'excentricité temporale par rapport au centre de la fovéa et ceci avec les 2 dispositifs d'imagerie. Au total, 6 images par oeil et par dispositif sont donc réalisées. La comparaison est fondée sur un système de notation basé sur la visibilité des photorécepteurs sur une échelle de 5 grades (notés de 5 pour les meilleures à 1 pour les moins bonnes). La notation est faite par 4 observateurs. Les résultats ont montré qu'en moyenne les images réalisées avec le système de diamètre de pupille d'entrée de 5 mm ont des notes de 1 grade meilleures que celles réalisées avec le système de pupille d'entrée de 7,5 mm. Comparées une à une (la meilleure des 3 images pour le même oeil, même position dans la rétine) les 19 images réalisées avec le système de diamètre de pupille d'entrée 5 mm sont meilleures dans 15 cas sur 19, aussi bonnes dans 2 cas sur 19 et moins bonnes dans 2 cas sur 19 que celle réalisées avec le système de pupille d'entrée de 7,5mm.

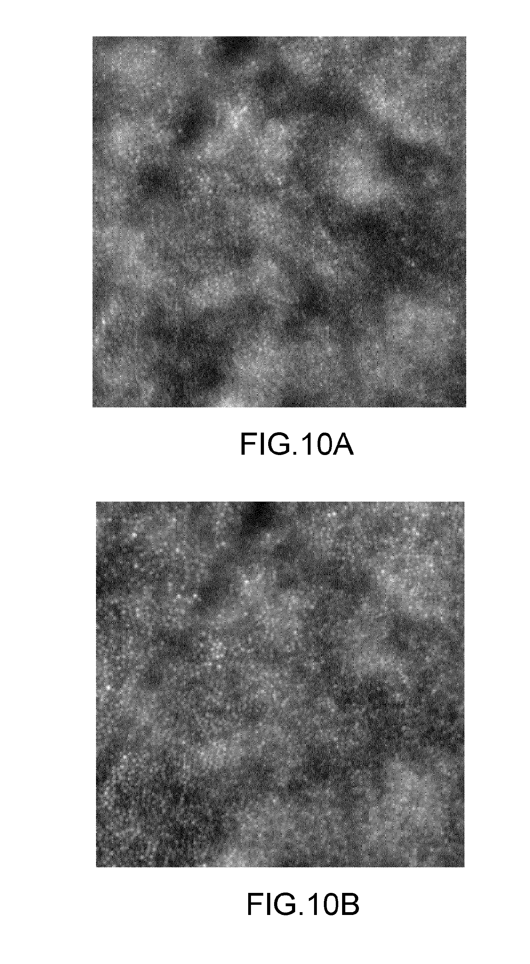

Les figures 10A et 10B illustrent le résultat de cette étude. Les 2 images présentées ont été réalisées sur le même oeil (personne âgée de 58 ans) et exactement au même endroit sur la rétine, à une excentricité de 2 degrés temporal, avec des dispositifs d'imagerie rétinienne dont le système d'imagerie de la rétine sur le détecteur présentent des pupilles d'entrée de respectivement 7,5 et 5 mm. Elles présentent 1 grade d'écart sur l'échelle de notation en faveur du système de pupille d'entrée 5 mm. Elles sont donc représentatives de l'écart moyen constaté entre les 2 dispositifs.

Bien que décrite à travers un certain nombre d'exemples de réalisation détaillés, le dispositif d'imagerie rétinienne et la méthode selon l'invention comprennent différentes variantes, modifications et perfectionnements qui apparaîtront de façon évidente à l'homme de l'art, étant entendu que ces différentes variantes, modifications et perfectionnements font partie de la portée de l'invention, telle que définie par les revendications qui suivent.

## Revendications

1. Dispositif d'imagerie rétinienne à haute résolution comprenant

    - Au moins une source d'émission ($LS_r$) d'un faisceau lumineux pour l'illumination de la rétine d'un oeil (10) d'un sujet, émettant dans une plage de longueurs d'onde d'imagerie donnée,
    - Une voie d'imagerie de la rétine comprenant un dispositif (12) de détection apte à détecter des structures de fréquence spatiale de 250 cycles/mm mesurées dans le plan de la rétine et un système optique d'imagerie (16),
- une voie d'analyse comprenant un dispositif (15) de mesure de défauts optiques avec un plan d'analyse destiné à recevoir un ensemble de rayons lumineux rétrodiffusés par la rétine et des moyens de conjugaison optique dudit plan d'analyse avec un plan prédéterminé (17) dans l'espace d'entrée dudit système optique d'imagerie,
- un dispositif (14) de correction comprenant un plan de correction et destiné à corriger dans ledit plan de correction les rayons lumineux issus de ladite source d'émission (LS$_r$) et rétrodiffusés par la rétine en fonction des défauts optiques mesurés par le dispositif (15) de mesure de défauts optiques, ledit plan de correction étant conjugué optiquement avec ledit plan prédéterminé (17) dans l'espace d'entrée du système optique d'imagerie de la voie d'imagerie, le dispositif d'imagerie rétinienne étant **caractérisé en ce que** :

la pupille d'entrée du dudit système optique d'imagerie présente un diamètre compris entre une première valeur $\Phi_{min}$ et une seconde valeur $\Phi_{max}$, la première valeur étant définie pour permettre la détection par ledit dispositif de détection (12) à la longueur d'onde centrale de ladite plage de longueurs d'onde d'imagerie de structures de la rétine présentant une fréquence spatiale de 250 cycles par millimètre, et la seconde valeur étant inférieure à 5,75 mm.

2. Dispositif selon la revendication 1, dans lequel ladite pupille d'entrée du système d'imagerie est positionnée dans ledit plan prédéterminé (17) de l'espace d'entrée dudit système d'imagerie.

3. Dispositif selon l'une quelconque des revendications précédentes, dans lequel le dispositif de correction comprend un miroir déformable dont la pupille définit la pupille physique du système d'imagerie (16).

4. Dispositif selon l'une quelconque des revendications précédentes, dans lequel ladite première valeur $\Phi_{min}$ est définie en fonction de ladite longueur d'onde centrale de la plage de longueurs d'onde d'imagerie pour obtenir un contraste théorique du système d'imagerie supérieur à 5% à ladite fréquence spatiale de 250 cycles par millimètre.

5. Dispositif selon la revendication 4, dans lequel ladite première valeur $\Phi_{min}$ est donnée par la relation $\Phi_{min} = 5000 \times \lambda$ où $\lambda$ est ladite longueur d'onde centrale de la plage de longueurs d'onde d'imagerie.

6. Dispositif selon l'une quelconque des revendications précédentes, dans lequel ladite longueur d'onde centrale de la plage de longueurs d'onde d'imagerie est comprise entre 750 et 1100 nm et le diamètre de la pupille d'entrée du système d'imagerie compris entre 3,75 mm et 5,75 mm.

7. Dispositif selon l'une quelconque des revendications 1 à 5, dans lequel ladite longueur d'onde centrale de la plage de longueurs d'onde d'imagerie est comprise entre 500 et 750 nm et le diamètre de la pupille d'entrée du système d'imagerie compris entre 2,5 mm et 5,25 mm.

8. Dispositif selon l'une quelconque des revendications 1 à 5, dans lequel ladite longueur d'onde centrale de la plage de longueurs d'onde d'imagerie est comprise entre 350 et 500 nm et le diamètre de la pupille d'entrée du système d'imagerie compris entre 1,75 mm et 4,25 mm.

9. Dispositif selon l'une quelconque des revendications précédentes, **caractérisé en ce que** le dispositif est de type plein champ, ladite source d'illumination (LS$_r$) étant une source étendue permettant d'illuminer la rétine avec un champ donné, et le dispositif de détection comprenant un détecteur matriciel, le dispositif comprenant en outre une seconde source d'illumination (LS$_a$) de la rétine émettant dans une plage de longueurs d'onde d'analyse donnée, ponctuelle, pour l'analyse des défauts optiques par ledit dispositif de mesure de défauts optiques.

10. Dispositif selon l'une quelconque des revendications 1 à 8, **caractérisé en ce que** le dispositif est de type AOSLO, ladite source d'illumination étant une source ponctuelle permettant d'illuminer la rétine avec un faisceau d'illumination quasi ponctuel et le dispositif de détection comprenant un système de détection confocale, le dispositif comprenant en outre un système de balayage dudit faisceau d'illumination sur la rétine.

11. Dispositif selon l'une quelconque des revendications 1 à 8, **caractérisé en ce que** le dispositif est de type OCT, ladite source d'illumination étant une source ponctuelle permettant d'illuminer la rétine avec un faisceau d'illumination quasi ponctuel et le dispositif de détection comprenant un interféromètre, le dispositif comprenant en outre un système de balayage dudit faisceau d'illumination sur la rétine.

**12.** Dispositif selon l'une des revendications précédentes, dans lequel le dispositif de mesure des défauts optiques (15) est un analyseur de type Shack-Hartmann.

**13.** Méthode d'imagerie rétinienne haute résolution, comprenant

- l'émission d'au moins un faisceau lumineux pour l'illumination de la rétine d'un oeil (10) d'un sujet, dans une plage de longueurs d'onde d'imagerie donnée, au moyen d'une source d'émission lumineuse,
- la formation d'une image d'au moins une partie de la rétine illuminée par ledit faisceau lumineux émis dans ladite plage de longueurs d'onde d'imagerie sur un plan de détection d'un dispositif de détection (12) apte à détecter des structures de fréquence spatiale de 250 cycles/mm mesurées dans le plan de la rétine et au moyen d'un système optique d'imagerie (16) de pupille d'entrée de diamètre donné,
- la mesure de défauts optiques par l'analyse dans un plan d'analyse donné des défauts optiques de rayons lumineux rétrodiffusés par la rétine, ledit plan d'analyse étant conjugué optiquement avec un plan prédéterminé de l'oeil,
- la correction dans un plan de correction donné des rayons lumineux issus de ladite source d'émission et rétrodiffusés par la rétine en fonction des défauts optiques mesurés, ledit plan de correction étant conjugué optiquement avec ledit plan prédéterminé (17) de l'oeil, la méthode étant **caractérisée en ce que** :

le diamètre de la pupille d'entrée du dudit système optique d'imagerie est compris entre une première valeur $\Phi_{min}$ et une seconde valeur $\Phi_{max}$, la première valeur étant définie pour permettre la détection par ledit dispositif de détection (12) à la longueur d'onde centrale de ladite plage de longueurs d'onde d'imagerie de structures de la rétine présentant une fréquence spatiale de 250 cycles par millimètre, et la seconde valeur étant inférieure à 5,75 mm.

**14.** Méthode d'imagerie rétinienne selon la revendication 13 de type plein champ, comprenant en outre l'émission d'un faisceau lumineux d'analyse dans une plage de longueurs d'onde d'analyse donnée pour l'analyse des défauts optiques, et dans laquelle le faisceau lumineux à ladite longueur d'onde centrale de la plage de longueurs d'onde d'imagerie permet l'illumination de la rétine avec un champ donné et la formation de l'image dudit champ de la rétine est faite au moyen d'un détecteur matriciel.

**15.** Méthode d'imagerie rétinienne selon la revendication 13 de type AOSLO, comprenant en outre un balayage dudit faisceau d'illumination de la rétine et une détection confocale.

**16.** Méthode d'imagerie rétinienne selon la revendication 13 de type OCT, comprenant en outre une détection interférométrique.

**Patentansprüche**

**1.** Vorrichtung zur hochauflösenden Netzhautbildgebung, umfassend

- mindestens eine Quelle (LS$_r$) zum Aussenden eines Lichtstrahls zur Beleuchtung der Netzhaut eines Auges (10) eines Probanden, wobei das Aussenden in einem gegebenen Bereich von Bildgebungswellenlängen erfolgt,
- einen Weg zur Bildgebung der Netzhaut, welcher eine Detektionsvorrichtung (12), die dazu geeignet ist, Strukturen mit einer Ortsfrequenz von 250 Zyklen/mm zu erfassen, wobei die Messung derselben in der Netzhautebene erfolgt, sowie ein optisches Bildgebungssystem (16) umfasst,
- einen Untersuchungsweg, der eine Vorrichtung (15) zur Messung optischer Fehler mit einer Untersuchungsebene, die dazu bestimmt ist, eine Gesamtheit von Lichtstrahlen zu empfangen, welche von der Netzhaut zurückgestreut werden, sowie Mittel zur optischen Verbindung der Untersuchungsebene mit einer vorbestimmten Ebene (17), die sich im Eintrittsraum des optischen Bildgebungssystems befindet, umfasst,
- eine Korrekturvorrichtung (14), die eine Korrekturebene umfasst und dazu bestimmt ist, die Lichtstrahlen, welche aus der Aussendequelle (LS$_r$) stammen und von der Netzhaut zurückgestreut werden, in Abhängigkeit von den optischen Fehlern, die von der Vorrichtung (15) zur Messung optischer Fehler gemessen wurden, einer Korrektur zu unterziehen, wobei die Korrekturebene mit der vorbestimmten Ebene (17), die sich im Eintrittsraum des optischen Bildgebungssystems befindet, optisch verbunden ist, wobei Vorrichtung zur Netzhautbildgebung **dadurch gekennzeichnet ist, dass**:
- die Eintrittspupille des optischen Bildgebungssystems einen Durchmesser im Bereich von einem ersten Wert $\Phi_{min}$ bis zu einem zweiten Wert $\Phi_{max}$ aufweist, wobei der erste Wert derart festgelegt ist, dass er es ermöglicht, Strukturen der Netzhaut, die eine Ortsfrequenz von 250 Zyklen pro Millimeter aufweisen, mittels der Detektionsvorrichtung (12) bei der mittigen Wellenlänge des Bereichs von Bildgebungswellenlängen zu erfassen, und wobei der zweite Wert geringer als 5,75 mm ist.

2. Vorrichtung nach Anspruch 1, wobei die Eintrittspupille des Bildgebungssystems in der vorbestimmten Ebene (17) des Eintrittsraums des Bildgebungssystems angeordnet ist.

3. Vorrichtung nach einem beliebigen der vorhergehenden Ansprüche, wobei die Korrekturvorrichtung einen verformbaren Spiegel umfasst, dessen Pupille die physikalische Pupille des Bildgebungssystems (16) bestimmt.

4. Vorrichtung nach einem beliebigen der vorhergehenden Ansprüche, wobei der erste Wert Φmin in Abhängigkeit von der mittigen Wellenlänge des Bereichs von Bildgebungswellenlängen festgelegt ist, um einen theoretischen Kontrast des Bildgebungssystems zu erzielen, der die Ortsfrequenz von 250 Zyklen pro Millimeter um 5 % übertrifft.

5. Vorrichtung nach Anspruch 4, wobei sich der erste Wert Φmin aus der Beziehung $\Phi min = 5000 \times \lambda$ ergibt, wobei $\lambda$ die mittige Wellenlänge des Bereichs von Bildgebungswellenlängen ist.

6. Vorrichtung nach einem beliebigen der vorhergehenden Ansprüche, wobei die mittige Wellenlänge des Bereichs von Bildgebungswellenlängen 750 bis 1.100 nm beträgt und der Durchmesser der Eintrittspupille des Bildgebungssystems 3,75 mm bis 5,75 mm beträgt.

7. Vorrichtung nach einem beliebigen der Ansprüche 1 bis 5, wobei die mittige Wellenlänge des Bereichs von Bildgebungswellenlängen 500 bis 750 nm beträgt und der Durchmesser der Eintrittspupille des Bildgebungssystems 2,5 mm bis 5,25 mm beträgt.

8. Vorrichtung nach einem beliebigen der Ansprüche 1 bis 5, wobei die mittige Wellenlänge des Bereichs von Bildgebungswellenlängen 350 bis 500 nm beträgt und der Durchmesser der Eintrittspupille des Bildgebungssystems 1,75 mm bis 4,25 mm beträgt.

9. Vorrichtung nach einem beliebigen der vorhergehenden Ansprüche, **dadurch gekennzeichnet, dass** die Vorrichtung der Bauart nach ein Vollfeldgerät ist, wobei es sich bei der Beleuchtungsquelle ($LS_r$) um eine ausgedehnte Quelle handelt, die es ermöglicht, die Netzhaut mit einem gegebenen Feld zu beleuchten, wobei die Detektionsvorrichtung weiterhin einen Matrixdetektor umfasst, wobei die Vorrichtung darüber hinaus eine zweite Quelle ($LS_a$) zur Beleuchtung der Netzhaut umfasst, welche in einem gegebenen Bereich von Untersuchungswellenlängen aussendet, wobei sie von punktförmiger Beschaffenheit ist, um die optischen Fehler mittels der Vorrichtung zur Messung optischer Fehler zu untersuchen.

10. Vorrichtung nach einem beliebigen der Ansprüche 1 bis 8, **dadurch gekennzeichnet, dass** die Vorrichtung von der Bauart AOSLO ist, wobei es sich bei der Beleuchtungsquelle um eine punktförmige Quelle handelt, die es ermöglicht, die Netzhaut mit einem nahezu punktförmigen Beleuchtungsstrahl zu beleuchten, und wobei die Detektionsvorrichtung ein System zur konfokalen Detektion umfasst, wobei die Vorrichtung darüber hinaus ein System zum Abtasten der Netzhaut mit dem Beleuchtungsstrahl umfasst.

11. Vorrichtung nach einem beliebigen der Ansprüche 1 bis 8, **dadurch gekennzeichnet, dass** die Vorrichtung von der Bauart OCT ist, wobei es sich bei der Beleuchtungsquelle um eine punktförmige Quelle handelt, die es ermöglicht, die Netzhaut mit einem nahezu punktförmigen Beleuchtungsstrahl zu beleuchten, und wobei die Detektionsvorrichtung ein Interferometer umfasst, wobei die Vorrichtung darüber hinaus ein System zum Abtasten der Netzhaut mit dem Beleuchtungsstrahl umfasst.

12. Vorrichtung nach einem der vorhergehenden Ansprüche, wobei es sich bei der Vorrichtung (15) zur Messung der optischen Fehler um einen Sensor des Typs Shack-Hartmann handelt.

13. Verfahren zur hochauflösenden Netzhautbildgebung, das Folgendes umfasst

- Aussenden mindestens eines Lichtstrahls zur Beleuchtung der Netzhaut eines Auges (10) eines Probanden, in einem gegebenen Bereich von Bildgebungswellenlängen erfolgt, mittels einer lichtaussendenden Quelle,
- Erzeugen einer Abbildung mindestens eines Teils der Netzhaut, die von dem Lichtstrahl beleuchtet wird, welcher in dem Bereich von Bildgebungswellenlängen ausgesendet wird, auf einer Detektionsebene einer Detektionsvorrichtung (12), die dazu befähigt ist, Strukturen mit einer Ortsfrequenz von 250 Zyklen/mm zu erfassen, wobei diese in der Netzhautebene gemessen werden, und zwar mittels eines optischen Bildgebungssystems (16) mit einer Eintrittspupille mit gegebenem Durchmesser,

- Messen von optischen Fehlern, indem die optischen Fehler von Lichtstrahlen, welche von der Netzhaut zurückgestreut werden, in einer gegebenen Untersuchungsebene untersucht werden, wobei die Untersuchungsebene optisch mit einer vorbestimmten Ebene des Auges verbunden ist,

- Korrigieren der Lichtstrahlen, die aus der aussendenden Quelle stammen und von der Netzhaut zurückgestreut wurden, in einer gegebenen Korrekturebene, in Abhängigkeit von den gemessenen optischen Fehlern, wobei die Korrekturebene optisch mit der vorbestimmten Ebene (17) des Auges verbunden ist, wobei das Verfahren **dadurch gekennzeichnet ist, dass**:

- der Durchmesser der Eintrittspupille des optischen Bildgebungssystems im Bereich von einem ersten Wert $\Phi_{min}$ bis zu einem zweiten Wert $\Phi_{max}$ aufweist, wobei der erste Wert derart festgelegt ist, dass er es ermöglicht, Strukturen der Netzhaut, die eine Ortsfrequenz von 250 Zyklen pro Millimeter aufweisen, mittels der Detektionsvorrichtung (12) bei der mittigen Wellenlänge des Bereichs von Bildgebungswellenlängen zu erfassen, und wobei der zweite Wert geringer als 5,75 mm ist.

14. Verfahren zur hochauflösenden Netzhautbildgebung nach Anspruch 13 vom Vollfeldtyp, wobei dieses darüber hinaus das Aussenden eines Untersuchungslichtstrahls in einem gegebenen Bereich von Untersuchungswellenlängen umfasst, um die optischen Fehler zu untersuchen, und wobei es der Lichtstrahl bei der mittigen Wellenlänge des Bereichs von Bildgebungswellenlängen ermöglicht, die Netzhaut mit einem gegebenen Feld zu beleuchten, und wobei die Abbildung des Feldes der Netzhaut mittels eines Matrixdetektors erzeugt wird.

15. Verfahren zur hochauflösenden Netzhautbildgebung nach Anspruch 13 vom Typ AOSLO, wobei dieses darüber hinaus ein Abtastbewegung des Strahls zur Beleuchtung der Netzhaut sowie eine konfokale Detektion umfasst.

16. Verfahren zur hochauflösenden Netzhautbildgebung nach Anspruch 13 vom Typ OCT, wobei dieses darüber hinaus eine interferometrische Detektion umfasst.

**Claims**

1. A high-resolution retinal imaging device comprising

- at least one emission source ($LS_r$) for emitting a light beam for the illumination of the retina of an eye (10) of a subject, emitting in a given range of imaging wavelengths,
- a retina imaging path comprising a detection device (12) capable of detecting spatial-frequency structures of 250 cycles/mm measured in the plane of the retina and an optical imaging system (16),
- an analysis path comprising a device (15) for measuring optical defects with an analysis plane intended to receive a set of light rays backscattered by the retina and means for optically conjugating said analysis plane with a predetermined plane (17) in the input space of said optical imaging system,
a correction device (14) comprising a correction plane and intended to correct in said correction plane the light rays from said emission source ($LS_r$) and backscattered by the retina as a function of the optical defects measured by the device (15) for measuring optical defects, said correction plane being optically conjugated with said predetermined plane (17) in the input space of the optical imaging system of the imaging path, the retinal imaging device being **characterized in that**:

the input pupil of said optical imaging system has a diameter between a first value $\Phi_{min}$ and a second value $\Phi_{max}$, the first value being defined to allow for the detection by said detection device (12) at the central wavelength of said range of imaging wavelengths of structures of the retina having a spatial frequency of 250 cycles per millimeter, and the second value being less than 5.75 mm.

2. The device as claimed in claim 1, in which said input pupil of the imaging system is positioned in said predetermined plane (17) of the input space of said imaging system.

3. The device as claimed in any one of the preceding claims, in which the correction device comprises a deformable mirror, the pupil of which defines the physical pupil of the imaging system (16).

4. The device as claimed in any one of the preceding claims, in which said first value $\Phi_{min}$ is defined as a function of said central wavelength of the range of imaging wavelengths to obtain a theoretical contrast of the imaging system greater than 5% at said spatial frequency of 250 cycles per millimeter.

5. The device as claimed in claim 4, in which said first value $\Phi_{min}$ is given by the relationship $\Phi_{min} = 5000 \times \lambda$ where $\lambda$ is said central wavelength of the range of imaging wavelengths.

6. The device as claimed in any one of the preceding claims, in which said central wavelength of the range of imaging wavelengths is between 750 and 1100 nm and the diameter of the input pupil of the imaging system is between 3.75 mm and 5.75 mm.

7. The device as claimed in any one of claims 1 to 5, in which said central wavelength of the range of imaging wavelengths is between 500 and 750 nm and the diameter of the input pupil of the imaging system is between 2.5 mm and 5.25 mm.

8. The device as claimed in any one of claims 1 to 5, in which said central wavelength of the range of imaging wavelengths is between 350 and 500 nm and the diameter of the input pupil of the imaging system is between 1.75 mm and 4.25 mm.

9. The device as claimed in any one of the preceding claims, **characterized in that** the device is of the full-field type, said emission source ($LS_r$) being an extended source making it possible to illuminate the retina with a given field, and the detection device comprising a matrix detector, the device also comprising a second emission source ($LS_a$) for illuminating the retina emitting in a given discrete range of analysis wavelengths, for the analysis of the optical defects by said device for measuring optical defects.

10. The device as claimed in any one of claims 1 to 8, **characterized in that** the device is of the AOSLO type, said emission source being a point source making it possible to illuminate the retina with a quasi-point illumination beam and the detection device comprising a confocal detection system, the device also comprising a system for scanning said illumination beam on the retina.

11. The device as claimed in any one of claims 1 to 8, **characterized in that** the device is of the OCT type, said emission source being a point source making it possible to illuminate the retina with a quasi-point illumination beam and the detection device comprising an interferometer, the device also comprising a system for scanning said illumination beam on the retina.

12. The device as claimed in any one of the preceding claims, in which the device for measuring optical defects (15) is an analyzer of the Shack-Hartmann type.

13. A high-resolution retinal imaging method, comprising

- the emission of at least one light beam for the illumination of the retina of an eye (10) of a subject, in a given range of imaging wavelengths, by means of a light-emission source,
- the formation of an image of at least a part of the retina illuminated by said light beam emitted in said range of imaging wavelengths on a detection plane of a detection device (12) capable of detecting spatial-frequency structures of 250 cycles/mm measured in the plane of the retina and by means of an optical imaging system (16) with an input pupil of given diameter,
- the measurement of optical defects by the analysis in a given analysis plane of the optical defects of light rays backscattered by the retina, said analysis plane being optically conjugated with a predetermined plane of the eye,
- the correction in a given correction plane of the light rays from said emission source and backscattered by the retina as a function of the measured optical defects, said correction plane being optically conjugated with said predetermined plane (17) of the eye, the method being **characterized in that**:

the diameter of the input pupil of said optical imaging system is between a first value $\Phi_{min}$ and a second value $\Phi_{max}$, the first value being defined to allow for the detection by said detection device (12) at the central wavelength of said range of imaging wavelengths of structures of the retina exhibiting a spatial frequency of 250 cycles per millimeter, and the second value being less than 5.75 mm.

14. The retinal imaging method as claimed in claim 13 of the full-field type, also comprising the emission of an analysis light beam in a given range of analysis wavelengths for the analysis of the optical defects, and in which the light beam at said central wavelength of the range of imaging wavelengths allows for the illumination of the retina with a given field and the formation of the image of said field of the retina is done by means of a matrix detector.

15. The retinal imaging method as claimed in claim 13, of the AOSLO type, also comprising a scanning of said illumination beam of the retina and a confocal detection.

16. The retinal imaging method as claimed in claim 13, of the OCT type, also comprising an interferometric detection.

EP 2 670 294 B1

FIG.1A

FIG.1B

FIG.1C

FIG.2

A-A

FIG.3A

FIG.3B

FIG.4

FIG.5

Plein champ  850 nm

FIG.6A

Plein champ 550 nm

FIG.6B

Diamètre du trou confocal
(rapporté au diamètre du disque d'Airy)

FIG.7A

SLO 850 nm

Diamètre (mm)

FIG.7B

Plein champ  750 nm

FIG.8A

Plein champ  500 nm

FIG.8B

Plein champ  350 nm

FIG.8C

SLO 1100 nm

FIG.8D

SLO 750 nm

FIG.8E

SLO 500 nm

FIG.8F

FIG.9

FIG.10A

FIG.10B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20070258045 A

**Littérature non-brevet citée dans la description**

- **A. ROORDA et al.** Adaptive optics scanning laser ophtalmoscopy. *Optics express,* 2002, vol. 10 (9), 405
- **R. ZAWADZKI.** Adaptive-optics optical coherence tomography for high resolution and high speed 3D retinal in vivo imaging. *Optics Express,* 2005, vol. 13 (21), 8532
- **A. ROORDA.** Adaptive Optics Ophthalmoscopy. *Journal of Refractive Surgery,* Septembre 2000, vol. 16
- **H. HOFER et al.** Improvements in retinal image quality with dynamic correction of the eye's aberrations. *Optics Express,* 2001, vol. 8 (11), 631-643
- **JAN VAN DE KRAATS ; DIRK VAN NORREN.** Directional and nondirectional specral reflection from the human fovea. *Journal of Biomedical Optics,* Mars 2008, vol. 13 (2), 024010